# EUROPÄISCHE PATENTSCHRIFT

(11) **EP 1 863 535 B1**
(45) Veröffentlichungstag und Bekanntmachung des Hinweises auf die Patenterteilung: **07.06.2017**
(21) Anmeldenummer: 06707279.3
(22) Anmeldetag: 27.02.2006
(51) Int. Cl.: A61K 47/22, A61K 38/15, A61K 31/5513, A61K 9/00, A01N 43/90, A01N 43/84, A01P 5/00, A61P 33/00

(54) **ENDOPARASITIZIDE MITTEL**
ENDOPARASITICIDE
PRODUIT ENDOPARASITICIDE

(30) Priorität: 11.03.2005 DE 102005011779
(43) Veröffentlichungstag der Anmeldung: 12.12.2007
(73) Patentinhaber: Bayer Intellectual Property GmbH, 40789 Monheim (DE)
(72) Erfinder: KANIKANTI, Venkata-Rangarao, 51381 Leverkusen (DE); TRÄUBEL, Michael, 50733 Köln (DE)
(74) Vertreter: BIP Patents
(86) Internationale Anmeldenummer: PCT/EP2006/001759
(87) Internationale Veröffentlichungsnummer: WO 2006/094664

(56) Entgegenhaltungen:
- EP-A- 0 267 404
- EP-A- 0 662 326
- WO-A-01/62268
- WO-A-2004/089239
- WO-A-2005/055973

## Beschreibung

Die vorliegende Erfindung betrifft äußerlich applizierbare Mittel, enthaltend Emodepside und Praziquantel oder Epsiprantel sowie 1,2-Isopropylidenglycerol, ihre Herstellung und ihre Verwendung zur Bekämpfung von Endoparasiten.

Der anthelmintische Wirkstoff Praziquantel (US P 4 001 411) und der strukturell verwandte Wirkstoff Epsiprantel (US P 4 661 489) werden üblicherweise oral verabreicht, siehe z. B. DE-A-199 41 024, WO 98/03157, US 2002/0081292 A1 und WO 97/25976. Bei topischer Applikation von Endoparasitiziden muß der Wirkstoff durch die Haut in den Blutkreislauf gelangen, um die betreffenden Endoparasiten zu erreichen. Da Praziquantel und Epsiprantel sich wenig zur transdermalen Applikation eignen, ist bei diesen Wirkstoffen die topische transdermale Applikationsform aufgrund der zu erwartenden Schwierigkeiten unüblich. Ein Mittel zur dermalen Behandlung von Wurmerkrankungen mit Praziquantel wird in EP-A-267 404 beschrieben. Die Anwendung dieses Mittels ist jedoch auf die Katze beschränkt, bei der wirksame transdermale Applikation in der Regel wesentlich leichter zu erreichen ist als z. B. bei Hunden.

WO 01/60380 (Phoenix Scientific, Inc.) offenbart parasitizide Formulierungen zur Injektion oder zur Pour-on-Anwendung, die ein Pyrrolidon-Lösungsmittel, ein weiteres Lösungsmittel und einen parasitiziden Wirkstoff enthalten können. In der umfangreichen Liste der Wirkstoffe wird unter anderem auch Praziquantel genannt.

EP-A-1 308 163 (Wyeth) offenbart endoparasitizide Mittel in Gelform, die Moxidectin, Praziquantel, Benzylalkohol, Ethanol, kolloidales Siliziumdioxid, ein Tensid und ein Öl enthalten.

WO 95/23590 (Bomac Laboratories) offenbart ein kompliziertes Verfahren zur Herstellung von anthelmintischen Mitteln zur dermalen Applikation. Die Mittel enthalten einen Träger, einen Emulgator, ein Öl und ein Verdünnungsmittel. Als Wirkstoffe kommen vor allem Benzimidazole in Frage, unter anderem werden jedoch auch makrocyclische Lactone und Praziquantel genannt.

WO 02/094288 beschreibt ein Arzneimittel für Tiere, das ein Avermectin-Oxim-Derivat, insbesondere Selamectin, in Kombination mit Praziquantel enthält. Es wird auch die topische Applikation vorgeschlagen, entsprechende Formulierungen enthalten einen Di(C₂₋₄-glykol)mono(C₁₋₄-alkyl)ether und ein optionales hautverträgliches Lösungsmittel.

Das cyclische Depsipeptid PF1022 und seine Wirkung gegen Endoparasiten ist bekannt aus EP-A 382 173.

Weitere cyclische Depsipeptide und ihre endoparasitizide Wirkung sind Gegenstand der deutschen Patentanmeldungen EP-A 626 376; EP-A 626 375; EP-A 644 883.

Das anthelmintisch wirksame cyclische Depsipeptid Emodepsid ist aus WO 93/19053 bekannt.

Endoparasitizide Mittel enthaltend Praziquantel oder Epsiprantel und cyclische Depsipeptide sind in EP 662 326 beschrieben.

Gegenstand der WO 96/38165 sind endoparasitizide Mittel enthaltend Avermectine, Ivermectine, Milbemycine in Kombination mit cyclischen Depsipeptiden sowie gegebenenfalls Praziquantel oder Epsiprantel.

Mittel zur dermalen Applikation, die Depsipeptide wie z. B. Emodepsid, enthalten, sind u. a. in WO 01/62268 sowie in unserer Anmeldung WO 05/055973 beschrieben.

WO2004/089239 beschreibt veterinärmedizinische anthelmintische Formulierungen für die topische Anwendung, die als Wirkstoff unter anderen auch. Praziquantel enthalten können. Als eines von mehreren geeigneten Lösungsmitteln wird in dem Dokument auch Glycerinformal genannt.

Die Wirkungshöhe und/oder Wirkungsdauer der vorbekannten Mittel ist jedoch, insbesondere bei bestimmten Wirtstieren, gegen bestimmte Organismen und/oder bei niedrigen Anwendungskonzentrationen, nicht in allen Anwendungsgebieten völlig zufriedenstellend.

Wegen der vielfältigen Anforderungen an moderne Arzneimittel, beispielsweise was Wirkhöhe (z. B. Plasmakonzentration des Wirkstoffs), Wirkdauer, Wirkspektrum, Anwendungsspektrum, Toxizität, Kombination von Wirkstoffen, Kombination mit Formulierungshilfsmitteln und wegen des möglichen Auftretens von Resistenzen kann die Entwicklung neuer Arzneimittel nie als abgeschlossen betrachtet werden, und es besteht beständig ein hoher Bedarf an neuen Mitteln, die zumindest in Teilaspekten Vorteile gegenüber den bekannten Mitteln bringen.

Um dem Tierhalter eine möglichst einfache Applikation endoparasitizider Wirkstoffe zu ermöglichen, ist es zudem wünschenswert ein äußerlich applizierbares Mittel zu Verfügung zu stellen, wobei äußerliche Applikation im Rahmen dieser Anmeldung in der Regel Applikation auf die Haut oder das Fell von Tieren bedeutet.

Wie aus der Literatur bekannt ist, sind Moleküle mit Molekulargewichten>1000 u äußerst schlecht bei topischer Applikation durch die Haut zu penetrieren. Besonders schlecht penetrieren Peptide oder Proteine mit größeren Molekulargewichten (Cevc et al, Advanced Drug Delivery Reviews 18 (1996) 349-378; Bauer et al. Pharmazeutische Technologie, 1993, S. 364, Thieme Verlag; Gurny et al. Dermal and Transdermal Drug Delivery, 1993, S. 131, Wissenschaftliche Verlagsgesellschaft). Die Penetration ist jedoch bei endoparasitiziden Wirkstoffen Voraussetzung, da sie gegen Endoparasiten z. B. im Magen-Darm-Trakt zur Wirkung kommen sollen.

Obwohl im Stand der Technik vereinzelt vorgeschlagen wurde, Praziquantel bzw. cyclische Depsipeptide topisch zu applizieren, ist es dem Fachmann bekannt, dass diese Wirkstoffe hierfür sehr wenig geeignet sind und die bekannten Formulierungen daher nicht völlig befriedigend sind, insbesondere beispielsweise bei den sogenannten dosistreibenden Würmern wie dem Peitschenwurm *Trichuris vulpis* und/oder dem Bandwurm *Taenia canis*.

Es wurde nun ein endoparasitizides Mittel gefunden, das bei äußerlicher Anwendung sehr gute Wirkung gegen ein breites Spektrum von Endoparasiten zeigt und gleichzeitig eine gute Stabilität aufweist.

Gegenstand der vorliegenden Erfindung sind:
Mittel enthaltend als Wirkstoffe
   - Emodepside sowie
   - Praziquantel oder Epsiprantel
und als alleiniges Lösungsmittel 1,2-Isopropylidenglycerol, wobei die Mittel höchstens einen Wassergehalt von 1 Gew.-% aufweisen.

Gegenstand der Erfindung ist ferner ein Verfahren zur Herstellung solcher Mittel, bei dem man die Wirkstoffe mit dem Lösungsmittel und gegebenenfalls weiteren Hilfsstoffen vermischt.

Der INN Emodepside steht für die Verbindung mit dem systematischen Namen: Cyclo[(R)-lactoyl-N-methyl-1-leucyl-(R)-3-(p-morpholinophenyl)lactoyl-N-methyl-1-leucyl-(R)-lactoyl-N-methyl-1-leucyl-(R)-3-(p-morpholinophenyl)lactoyl-N-methyl-1-leucyl. Emodespide ist in WO 93/19053 beschrieben und hat die folgende Formel:

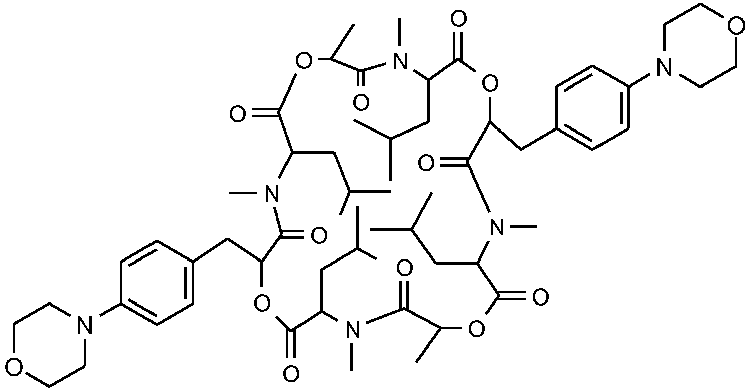

Praziquantel und Epsiprantel sind als Wirkstoffe gegen Endoparasiten lange bekannt (siehe z. B. US 4 661 489 für Epsiprantel und US 4 001 411 für Praziquantel). Praziquantel enthaltende Produkte sind z. B. unter der Bezeichnung Droncit® im Handel erhältlich. Im Rahmen dieser Erfindung ist die Verwendung von Praziquantel bevorzugt.

Erfindungsgemäß können sowohl reine Stereoisomere als auch Stereoisomerengemische eingesetzt werden. Soweit chemisch möglich ist auch die Verwendung von pharmazeutisch annehmbaren Salzen denkbar. Weiterhin können auch Prodrugs der Wirkstoffe in den Mitteln eingesetzt werden, wobei die eigentlichen Wirkstoffe nach Applikation der Prodrugs aus diesen freigesetzt werden.

Die erfindungsgemäßen Mittel eignen sich bei günstiger Warmblütertoxizität zur Bekämpfung von pathogenen Endoparasiten die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so dass durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen:
Mit Praziquantel oder Epsiprantel lassen sich vor allem folgende Endoparasiten bekämpfen:
   Aus der Ordnung der Pseudophyllidea z.B.: Diphyllobothrium spp., Spirometra spp., Schistocephalus spp., Ligula spp., Bothridium spp., Diphlogonoporus spp.

Aus der Ordnung der Cyclophyllidea z.B.: Mesocestoides spp., Anoplocephala spp., Paranoplocephala spp., Moniezia spp., Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.

Aus der Unterklasse der Monogenea z.B.: Gyrodactylus spp., Dactylogyrus spp., Polystoma spp.

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp.

Mit Emodepside lassen sich vor allem folgende Endoparasiten bekämpfen:
Aus der Ordnung der Enoplida z.B.: Trichuris spp., Capillaria spp., Trichomosoides spp., Trichinella spp.

Aus der Ordnung der Rhabditia z.B.: Micronema spp., Strongyloides spp.

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp.

Globocephalus spp., Syngamus spp., Cyathostoma spp., Metastrongylus spp., Dictyocaulus spp., Muellerius spp., protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp.

Aus der Ordnung der Oxyurida z.B.: Oxyuris spp., Enterobius spp., Passalurus spp., Syphacia spp., Aspiculuris spp., Heterakis spp.

Aus der Ordnung der Ascaridia z.B.: Ascaris spp., Toxascaris spp., Toxocara spp., Parascaris spp., Anisakis spp., Ascaridia spp.

Aus der Ordnung der Spirurida z.B.: Gnathostoma spp., Physaloptera spp., Thelazia spp., Gongylonema spp., Habronema spp., Parabronema spp., Draschia spp., Dracunculus spp.

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp.

Aus der Ordnung der Gigantorhynchida z.B.: Filicollis spp., Moniliformis spp., Macracanthorhynchus spp., Prosthenorchis spp.

Bei Einsatz geeigneter Wirkstoffkombinationen kann man das gesamte Spektrum an oben aufgeführten Endoparasiten abdecken. Besonders bevorzugt ist die Anwendung der erfindungsgemäßen Mittel gegen: *Toxocara cati, Toxascaris leonina, Ancylostoma tubaeforme, Dipylidium caninum, Taenia taeniaeformis* und *Echinococcus multilocularis*.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Strauße.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Ganz besonders bevorzugt ist die Anwendung bei der Katze.

Die erfindungsgemäßen Mittel sind vorzugsweise Fluide, z. B. Suspensionen, Emulsionen oder insbesondere Lösungen. Erfindungsgemäß wird 1,2-Isopropylidenglycerol als alleiniges Lösungsmittel eingesetzt.

Es hat sich überraschenderweise gezeigt, dass solche erfindungsgemäßen Mittel, die das Lösungsmittel 1,2-Isopropylidenglycerol - vorzugsweise in Konzentrationen von mindestens 60 Vol.-% - enthalten, bei äußerlicher Anwendung eine besonders gute Wirkung gegen Nematoden und Cestoden aufweisen, ohne dass so genannte Penetrationsenhancer verwendet werden müssen, wie sie üblicherweise im Stand der Technik empfohlen werden.

Um das Risiko der Hydrolyse von 1,2-Isopropylidenglycerol zu minimieren und um die Stabilität der Formulierungen deutlich zu verbessern, ist es empfehlenswert, den Wassergehalt der erfindungsgemäßen Mittel möglichst niedrig zu halten, üblicherweise sollte der Wassergehalt nicht größer als 1 Gew.%, bevorzugt nicht größer als 0,5 Gew.%, sein.

Ein Hydrolyseprodukt des 1,2-Isopropylidenglycerols ist Aceton, dessen Konzentration in Arzneimitteln gemäß internationaler Bestimmungen so niedrig wie möglich sein sollte. Weiterhin kann Aceton die Aufnahme der Wirkstoffe in den Körper beeinflussen. Die Veränderung dieser Eigenschaften z. B. durch Hydrolyse des 1,2-Isopropylidenglycerol während der Lagerung ist bei einem Arzneimittel nicht akzeptabel.

Der Lösungsmittelgehalt der erfindungsgemäßen Mittel beträgt üblicherweise 60 bis 96 Gew.-%, bevorzugt 70 bis 96 Gew.%, besonders bevorzugt 80 bis 90 Gew.-% bezogen auf das Gesamtgewicht des fertigen Mittels.

Es kann vorteilhaft sein, den erfindungsgemäßen Mitteln weitere in der Veterinärmedizin übliche Hilfsstoffe zuzufügen.

Bevorzugte Hilfsstoffe sind beispielsweise Oxidationsstabilisatoren wie z. B. Butylhydroxyanisol (BHA), Butylhydroxytoluol (BHT) und Ascorbinsäure. Diese können z. B. in Konzentrationen von 0,1 bis 1 Gew.%, bevorzugt 0,3 bis 0,7 Gew.-% bezogen auf das Gesamtgewicht des fertigen Mittels eingesetzt werden.

Weitere bevorzugte Hilfsstoffe sind Stabilisatoren, wie z. B. organische Säuren, insbesondere Milchsäure. Diese werden üblicherweise in Mengen von 1 bis 5 Gew.%, bevorzugt 1 bis 3 Gew.-% bezogen auf das Gesamtgewicht des fertigen Mittels eingesetzt.

Die erfindungsgemäßen Mittel können gegebenenfalls auch Synergisten oder weitere Wirkstoffe enthalten.

Anwendungsfertige Zubereitungen enthalten die Wirkstoffe üblicherweise in Konzentrationen von 0,1 bis 25 Gew.-%, bevorzugt von 0,1 bis 20 Gew.%, wobei für Emodepsid Konzentrationen von 0,5
bis 5 Gew.%, insbesondere 1 bis 3 Gew.-% und für Praziquantel oder Epsiprantel 1 bis 15 Gew.%, insbesondere 5 bis 10 Gew.-% bevorzugt sind.

Die Mittel werden durch Mischen der Komponenten in den entsprechenden Mengen in geeigneten Geräten hergestellt, vorzugsweise wird gemischt bis eine klare Lösung entsteht.

Im Allgemeinen hat es sich als vorteilhaft erwiesen, die erfindungsgemäßen Mittel so zu dosieren, dass pro Anwendung etwa 1 bis etwa 100 mg des jeweiligen Wirkstoffs je kg Körpergewicht verabreicht werden. Bevorzugt sind bei Emodepsid 1 bis 20 mg, insbesondere 1 bis 10 mg Wirkstoff je kg Körpergewicht und bei Praziquantel oder Epsiprantel 5 bis 50 mg, insbesondere 5 bis 20 mg Wirkstoff je kg Körpergewicht.

### Beispiel 1

100 g Lösung bestehend aus:

| | |
|---|---|
| 7,94 g | Praziquantel |
| 1,98 g | Emodepsid |
| 87,58 g | 1,2-Isopropylidenglycerol |
| 2,00 g | Milchsäure |
| 0,50 g | Butylhydroxyanisol |

Das Mittel gemäß Beispiel 1 erwies sich in klinischen Untersuchungen als sehr gut wirksam gegen Infektionen mit *Toxocara cati*, *Toxascaris leonina*, *Ancylostoma tubaeforme*, *Dipylidium caninum*, *Taenia taeniaeformis* und *Echinococcus multiocularis* bei Katzen.

## Patentansprüche

1. Mittel enthaltend als Wirkstoffe
- Emodepside sowie
- Praziquantel oder Epsiprantel
und als alleiniges Lösungsmittel 1,2-Isopropylidenglycerol, wobei die Mittel höchstens einen Wassergehalt von 1 Gew.-% aufweisen

2. Mittel gemäß Anspruch 1, **dadurch gekennzeichnet, dass** es als Wirkstoffe Emodepside und Praziquantel enthält.

3. Mittel gemäß einem der vorstehenden Ansprüche mit einem Lösungsmittelgehalt von 60 bis 96 Gew.%, bezogen auf das Gesamtgewicht des fertigen Mittels.

4. Mittel gemäß Anspruch 3 mit einem Lösungsmittelgehalt von 70 bis 96 Gew.%, bezogen auf das Gesamtgewicht des fertigen Mittels.

5. Mittel gemäß Anspruch 4 mit einem Lösungsmittelgehalt von 80 bis 90 Gew.-%, bezogen auf das Gesamtgewicht des fertigen Mittels.

6. Verfahren zur Herstellung der Mittel gemäß einem der vorstehenden Ansprüche, **dadurch gekennzeichnet, dass** man die Wirkstoffe mit dem Lösungsmittel und gegebenenfalls weiteren Hilfsstoffen vermischt.

7. Verwendung von Emodepside und Praziquantel oder Epsiprantel zur Herstellung von Mitteln, die 1,2-Isopropylidenglycerol als alleiniges Lösungsmittel enthalten, einen Wassergehalt von höchstens 1 Gew.-% aufweisen und sich für die äußerliche Anwendung eignen, zur Bekämpfung von Endoparasiten.

8. Mittel gemäß einem der Ansprüche 1 bis 5 zur Verwendung zur Bekämpfung von Endoparasiten.

9. Mittel gemäß Anspruch 8 zur Verwendung zur Bekämpfung von Endoparasiten ausgewählt aus: *Toxocara cati, Toxascaris leonina, Ancylostoma tubaeforme, Dipylidium caninum, Taenia taeniaeformis* und *Echinococcus multilocularis*.

10. Mittel gemäß Anspruch 8 zur Verwendung zur Bekämpfung von Endoparasiten bei Katzen.

## Claims

1. Compositions comprising, as active compounds,
- emodepside and
- praziquantel or epsiprantel
and, as only solvent, 1,2-isopropylideneglycerol, where the water content of the compositions is at most 1% by weight.

2. Composition according to Claim 1, **characterized in that** it comprises, as active compounds, emodepside and praziquantel.

3. Compositions according to either of the preceding claims, having a solvent content of from 60 to 96% by weight, based on the total weight of the finished composition.

4. Compositions according to Claim 3, having a solvent content of from 70 to 96% by weight, based on the total weight of the finished composition.

5. Compositions according to Claim 4, having a solvent content of from 80 to 90% by weight, based on the total weight of the finished composition.

6. Process for preparing the compositions according to any of the preceding claims, **characterized in that** the active compounds are mixed with the solvent and, if appropriate, further auxiliaries.

7. Use of emodepside and praziquantel or epsiprantel for preparing compositions comprising 1,2-isopropylideneglycerol as only solvent, having a water content of at most 1% by weight and being suitable for external application for controlling endoparasites.

8. Compositions according to any of Claims 1 to 5 for use for controlling endoparasites.

9. Compositions according to Claim 8 for use for controlling endoparasites selected from: *Toxocara cati, Toxascaris leonina, Ancylostoma tubaeforme, Dipylidium caninum, Taenia taeniaeformis* and *Echinococcus multilocularis*.

10. Compositions according to Claim 8 for use for controlling endoparasites in cats.

## Revendications

1. Agents contenant en tant que substances actives :
- de l'émodepside et
- du praziquantel ou de l'epsiprantel,
et en tant que solvant unique, du 1,2-isopropylidène-glycérol, les agents présentant au plus une teneur en eau de 1 % en poids.

2. Agent selon la revendication 1, **caractérisé en ce qu'**il contient en tant que substances actives de l'émodepside et du praziquantel.

3. Agent selon l'une quelconque des revendications précédentes, ayant une teneur en solvant de 60 à 96 % en poids, par rapport au poids total de l'agent fini.

4. Agent selon la revendication 3, ayant une teneur en solvant de 70 à 96 % en poids, par rapport au poids total de l'agent fini.

5. Agent selon la revendication 4, ayant une teneur en solvant de 80 à 90 % en poids, par rapport au poids total de l'agent fini.

6. Procédé de fabrication des agents selon l'une quelconque des revendications précédentes, **caractérisé en ce que** les substances actives sont mélangées avec le solvant et éventuellement d'autres adjuvants.

7. Utilisation d'émodepside et de praziquantel ou d'epsiprantel pour la fabrication d'agents qui contiennent du 1,2-isopropylidène-glycérol en tant que solvant unique, présentent une teneur en eau d'au plus 1 % en poids et sont appropriés pour une application extérieure pour lutter contre des endoparasites.

8. Agent selon l'une quelconque des revendications 1 à 5, destiné à une utilisation pour lutter contre des endoparasites.

9. Agent selon la revendication 8, destiné à une utilisation pour lutter contre des endoparasites choisis parmi : *Toxocara cati, Toxascaris leonina, Ancylostoma tubaeforme, Dipylidium caninum, Taenia taeniaeformis* et *Echinococcus multilocularis.*

10. Agent selon la revendication 8, destiné à une utilisation pour lutter contre des endoparasites chez les chats.
